# EUROPEAN PATENT SPECIFICATION

(11) **EP 3 427 731 B1**
(45) Date of publication and mention of the grant of the patent: **29.09.2021**
(21) Application number: 17763205.6
(22) Date of filing: 07.03.2017
(51) Int. Cl.: A61K 31/355, A61K 31/353, A23L 33/10, A23L 33/15, A61K 36/48, A61P 15/12, A61P 25/04, A61P 25/18, A61P 25/22, A61P 25/24, A61P 43/00

(54) **AMELIORATING AGENT FOR FEMALE-SPECIFIC PHYSICAL AND/OR MENTAL UNPLEASANT SYMPTOMS**
LINDERUNGSMITTEL FÜR FRAUENSPEZIFISCHE PHYSISCHE UND/ODER PSYCHISCHE UNANGENEHME SYMPTOME
AGENT D'AMÉLIORATION DE SYMPTÔMES DÉSAGRÉABLES PHYSIQUES ET/OU MENTAUX SPÉCIFIQUES À LA FEMME

(30) Priority: 08.03.2016 JP 2016044883
(43) Date of publication of application: 16.01.2019
(73) Proprietor: Otsuka Pharmaceutical Co., Ltd., Tokyo 101-8535 (JP)
(72) Inventor: UENO, Tomomi, Osaka-shi Osaka 540-0021 (JP); DEGUCHI, Tomoko, Osaka-shi Osaka 540-0021 (JP); ONODA, Atsuko, Osaka-shi Osaka 540-0021 (JP)
(74) Representative: dompatent von Kreisler Selting Werner - Partnerschaft von Patent- und Rechtsanwälten mbB
(86) International application number: PCT/JP2017/008892
(87) International publication number: WO 2017/154865

(56) References cited:
- EP-A1- 1 961 309
- WO-A2-2004/004638
- JP-A- 2003 300 879
- US-A- 5 498 631
- US-A1- 2005 080 109
- KATO ET AL: "The effects of y-tocopherol administration on pretibial edema in young women with premenstrual syndrome", ACTA MED. NAGASAKI., vol. 53, no. 3, 1 January 2008 (2008-01-01), pages 59-64, XP055415619,
- NAOKO ISHIWATA ET AL: "New equol supplement for relieving menopausal symptoms : Randomized, placebo-controlled trial of Japanese women", MENOPAUSE: THE JOURNAL OF THE NORTH AMERICAN MENOPAUSE SOCIETY, vol. 16, no. 1, 1 January 2009 (2009-01-01), pages 141-148, XP55614628, US ISSN: 1072-3714, DOI: 10.1097/gme.0b013e31818379fa
- T. ASO: "Equol Improves Menopausal Symptoms in Japanese Women", THE JOURNAL OF NUTRITION, vol. 140, no. 7, 19 May 2010 (2010-05-19), pages 1386S-1389S, XP55007310, ISSN: 0022-3166, DOI: 10.3945/jn.109.118307
- KATO, S. et al.: "The effects of y-tocopherol administration on pretibial edema in young women with premenstrual syndrome", Acta Med. Nagasaki., vol. 53, no. 3, 2008, pages 59-64, XP055415619, ISSN: 0001-6055
- LONDON , R.S. et al.: "Efficacy of alpha- tocopherol in the treatment of the premenstrual syndrome", J. Reprod. Med., vol. 32, no. 6, 1987, pages 400-404, XP009512413, ISSN: 0024-7758
- J IANG, Q. et al.: "gamma-tocopherol and its major metabolite, in contrast to alpha-tocopherol, inhibit cyclooxygenase activity in macrophages and epithelial cells", Proc. Natl. Acad. Sci. USA, vol. 97, no. 21, 2000, pages 11494-11499, XP002190542,
- JIANG, Q. et al.: "Long-chain carboxychromanols, metabolites of vitamin E, are potent inhibitors of cyclooxygenases", Proc. Natl. Acad. Sci. USA, vol. 105, no. 51, 2008, pages 20464-20469, XP055415626,
- KIM, H.W. et al.: "Intake of dietary soy isoflavones in relation to perimenstrual symptoms of Korean women living in the USA", Nurs. Health Sci., vol. 8, no. 2, 2006, pages 108-113, XP055415630,
- BRYANT, M. et al.: "Effect of consumption of soy isoflavones on behavioural, somatic and affective symptoms in women with premenstrual syndrome", Br. J. Nutr., vol. 93, no. 5, 2005, pages 731-739, XP055415631, ISSN: 0007-1145
- KANDA, Y. et al.: "The influence of equol production causes to mental complaints in premenstrual syndrome", Bulletin of Tokyo Healthcare University, vol. 2, no. 1, 2006, pages 39-42, XP009512455, ISSN: 1882-4455
- ASO, T.: "Equol improves menopausal symptoms in Japanese women", J. Nutr., vol. 140, no. 7, 2010, pages 1386S-1389S, XP055415631,

## Description

### Technical Field

The present invention relates to an ameliorator of a female-specific physical and/or mental unpleasant symptom, as defined in the claims.

### Background Art

Female-specific physical and/or mental unpleasant symptoms are unpleasant symptoms a cause of which is believed to be fluctuation of female hormones and the like. Female-specific physical and/or mental unpleasant symptoms include physical and/or mental unpleasant symptoms before menstruation (referred to as premenstrual syndrome, "PMS"), and physical and/or mental unpleasant symptoms during menstruation including menstrual cramps, and unpleasant symptoms caused by menstrual disorder, abnormal vaginal bleeding, autonomic ataxia, infertility, climacteric disorder, and the like.

For example, PMS mainly lasts for 3 to 10 days prior to menstruation and becomes less severe or disappears as menstruation starts. A possible cause of PMS is rapid decline of estrogen (estrogenic hormone) and progesterone (luteal hormone). However, it is believed that there are many other factors. The causes of PMS have not been clear yet. The symptoms of PMS include various symptoms such as sleepiness, pain in the low back, lower abdominal pain, headache, easy fatigability, shoulder stiffness, swelling, breast fullness, vertigo, rough skin, acne, melancholy, anxiousness, feeling of malaise, lethargy, impaired ability to concentrate, getting annoyed, etc.

The physical and/or mental unpleasant symptoms during menstruation include various symptoms such as physical pain, for example, lower abdominal pain, pain in the low back, headache, etc.; vague pain, for example, general feeling of malaise, lassitude, etc.; getting annoyed, lethargy, melancholy, getting irritable, nausea, stomachache, anorexia, diarrhea, vertigo, etc. It is also believed that the physical and/or mental unpleasant symptoms during menstruation are caused by many factors, and the causes have not been clear yet.

Since the causes of the female-specific physical and/or mental unpleasant symptoms are not clear and the characteristics and duration of the symptoms vary on individuals, a symptomatic therapy or a method of alleviating the symptoms by self-managing such as improvement of dietary life, habituation of fitness, relief of stress, etc. is generally adopted as the treatment and prevention of the female-specific physical and/or mental unpleasant symptoms.

For example, for symptomatic therapies for PMS, effectiveness of various components has been studied, and a therapeutic agent for PMS comprising soybean isoflavone aglycone, particularly daidzein has been reported (Patent Literature 1). Further, effect of equol which is produced by metabolism of daidzein on PMS has been studied (Non-patent Literature 1). For improving fluid retention symptoms such as swelling, use of γ-tocopherol is known, see e.g. Acta Med. Nagasaki, 2008, 59-64, to Kato et al. WO-A-2004/004638 reports the treatment and/or amelioration of inflammatory symptoms related to premenstrual syndrome (PMS), perimenopause or menopause with a medicament comprising tocopherol and an omega-3 PUFA. US-A-5498631 discloses an isoflavonoid,including equol, for use in treating symptoms of menopause or premenstrual syndrome.

### Citation List

### Patent Literatures

Patent Literature 1: JP-A 2003-300879

### Non-patent Literatures

Non-patent Literature 1: Bulletin of Tokyo Healthcare University, No.1, 2006, pages 39-42

### Summary of Invention

### Problems to be solved by the Invention

Many women experience female-specific physical and/or mental unpleasant symptoms, which may disturb daily life in severe cases. On the other hand, women advance into society, and they wish an ameliorator of the female-specific physical and/or mental unpleasant symptoms in a form easy to take even when they are away from home. However, an ameliorator having strong ameliorating effect on the female-specific physical and/or mental unpleasant symptoms and having a form easy to take has not been developed yet.

### Solutions to the Problems

Under the above-described circumstance, the present inventors intensively studied for developing an ameliorator of the female-specific physical and/or mental unpleasant symptoms in a form easy to take. As a result, they found that the female-specific physical and/or mental unpleasant symptoms, for example the physical and/or mental unpleasant symptoms before and/or during menstruation were ameliorated by γ-tocopherol and equol in combination. Thus the present invention was completed.

Specifically, the present invention provides:
[1] an ameliorator for use in a female-specific physical and/or mental unpleasant symptom, before and/or during menstruation, comprising γ-tocopherol and equol, which is to be ingested so as to provide a daily γ-tocopherol intake of 0.1 to 1000 mg and a daily equol intake of 0.1 to 50 mg, respectively.
[2] the ameliorator for use according to [1], which comprises the γ-tocopherol and the equol as distinct preparations from each other,
[3] the ameliorator for use according to [1], which comprises the γ-tocopherol and the equol in the same preparation,
[4] the ameliorator for use according to any one of [1] to [3], wherein the female-specific physical and/or mental unpleasant symptom before and/or during menstruation is at least one selected from the group consisting of pain, negative emotion, concentration ability, and change in behavior,
[5] the ameliorator for use according to any one of [1] to [4], wherein a combination ratio (total weight ratio) of the γ-tocopherol to the equol is 1:0.0001 to 1:500,
[6] the ameliorator for use according to any one of [1] to [5], which has the form of a beverage or food product
[7] a beverage or food product comprising the ameliorator for use according to any one of [1] to [5].

### Effect of the Invention

The present invention exerts one or more of the following effects.
(1) The present invention provides an ameliorator of a female-specific physical and/or mental unpleasant symptom (hereinafter referred to as "the ameliorator of the present invention").
(2) The present invention particularly provides an ameliorator of a physical and/or mental unpleasant symptom before and/or during menstruation.
(3) The present invention can provide the ameliorator of the present invention in a form easy to ingest.

### Brief Description of Drawings

[Fig. 1] Figure 1 shows results of VAS in administration tests of γ-tocopherol alone (reference). In the figure, "γ-Toc" means γ-tocopherol.
[Fig. 2] Figure 2 shows results of MDQ in administration tests of γ-tocopherol alone (reference) and combined administration tests of γ-tocopherol and equol. In the figure, "γ-Toc" means γ-tocopherol.
[Fig. 3] Figure 3 shows results of degrees of general amelioration in combined administration tests of γ-tocopherol and equol.

### Mode for carrying out the Invention

The "γ-tocopherol" is a kind of natural vitamin E contained in vegetable oils such as soybean oil, rapeseed oil, etc. Vitamin E is known to have excellent antioxidant effect to prevent oxidation of body fat. The γ-tocopherol as used herein is not particularly limited. As the γ-tocopherol, commercially available γ-tocopherol for pharmaceuticals or food materials may be used.

In the present invention, the daily intake of γ-tocopherol is 0.1 to 1000 mg, preferably 50 to 800 mg, or more preferably 60 to 450 mg, depending on the degree of a symptom to be ameliorated. The amount of γ-tocopherol contained in the ameliorator of the present invention can be appropriately determined within the range of the daily intake of γ-tocopherol, considering a dosage form etc. of the ameliorator of the present invention. One or plural ameliorators of the present invention may be ingested so that the daily intake is reached.

The "equol" is a metabolite produced by conversion of isoflavones mainly existing in the form of glycosides wherein the isoflavones are bound to saccharides with digestive enzymes in the body or enzymes produced from enterobacteria, etc., and has high estrogenic activity. In the present invention, is used equol that is a metabolite of at least one kind of daizeins selected from the group consisting of daizein glycosides, daizein, and dihydrodaizein. Examples of the daizein glycosides include daidzin, malonyldaidzin, acetyldaidzin, etc. The equol as used herein is not particularly limited, and may be one obtained by a synthesis method or one obtained by a fermentation method.

In the present invention, a soybean hypocotyl fermented product containing equol (hereinafter referred to as "the equol-containing soybean hypocotyl fermented product") can be also used. The equol-containing soybean hypocotyl fermented product is obtained by fermenting soybean hypocotyls with microorganisms having the ability to assimilate at least one kind of daizeins selected from the group consisting of daizein glycosides, daizein, and dihydrodaizein and then produce equol. The soybean hypocotyl is a part of a soybean which will become a budlet and a radicle when the soybean germinates, and is known to contain high levels of daizeins such as daizein glycosides, daizein, etc. The soybean hypocotyl as used herein is not limited as long as it contains daizeins. The variety and the production area of soybean, processing or unprocessing of soybean, the shape of soybean hypocotyl, etc. are not limited. For example, the soybean hypocotyl may be a hypocotyl separated from a raw soybean or a soybean subjected to heat, drying or steaming treatment or the like; a hypocotyl separated from an unprocessed soybean and then subjected to heat, drying or steaming treatment or the like; a powdery hypocotyl, or crushed or ground hypocotyl. The microorganism having the ability to produce equol as described above is not particularly limited as long as it is acceptable from the perspective of food hygiene and has the ability to produce equol. Examples of the microorganism include microorganisms belonging to genus Lactococcus such as Lactococcus garvieae; genus Streptococcus such as Streptococcus intermedius, and Streptococcus constellatus; and genus Bacteroides such as Bacteroides ovatus. For example, the microorganism having the ability to produce equol can be also isolated from feces of a human based on the equol-producing ability as an index. Further, Lactococcus 20-92 (FERM BP-10036), Streptococcus E-23-17 (FERM BP-6436), Streptococcus A6G225 (FERM BP-6437), and Bacteroides E-23-15 (FERM BP-6435) which are bacteria isolated and identified by the Applicant can be also used. The equol-containing soybean hypocotyl fermented product can be obtained by fermenting the soybean hypocotyl with the microorganism having the ability to produce equol under suitable conditions. The fermentation conditions are not particularly limited as long as equol is produced, and are appropriately determined by a person skilled in the art. For example, the equol-containing soybean hypocotyl fermented product obtained by the method described in WO2007/066655 can be used.

The equol-containing soybean hypocotyl fermented product contains, in addition to equol, various isoflavones including daizeins such as daidzin, malonyldaidzin, acetyldaidzin, daizein, and dihydrodaizein; genisteins such as genistin, malonylgenistin, acetylgenistin, genistein, and dihydrogenistein; and glyciteins such as glycitin, malonylglycitin, acetylglycitin, glycitein, and dihydroglycitein, and thus the equol-containing soybean hypocotyl fermented product can also exhibit useful physiological activities of these isoflavones. Further, the equol-containing soybean hypocotyl fermented product also contains saponin derived from the soybean hypocotyl, and thus the equol-containing soybean hypocotyl fermented product can also exhibit useful physiological activities (for example, antiviral activity etc.) of saponin. Therefore, when the ameliorator of the present invention comprises the equol-containing soybean hypocotyl fermented product, additional effects are expected. The equol-containing soybean hypocotyl fermented product has the composition of isoflavones other than equol which is different from the isoflavone composition of a soybean hypocotyl. Particularly, the soybean hypocotyl fermented product contains genisteins, which are feared to act as endocrine disrupters, at low rates.

The equol-containing soybean hypocotyl fermented product as used herein contains for example 1 to 20 mg, preferably 2 to 12 mg of equol; for example 0.1 to 30 mg, preferably 0.1 to 1.5 mg in total of daizeins; for example 0.05 to 2.5 mg, preferably 0.05 to 2 mg in total of genisteins; and for example 0.1 to 4 mg, preferably 2 to 3.5 mg in total of glyciteins. Composition rates of isoflavones in the equol-containing soybean hypocotyl fermented product are for example 30 to 75% (w/w), preferably 40 to 70%(w/w), or more preferably 45 to 70%(w/w) of equol, 1 to 20%(w/w), preferably 2 to 15%(w/w), or more preferably 4 to 12%(w/w) of daizeins, 0.1 to 20%(w/w), preferably 1 to 15%(w/w), or more preferably 1 to 10%(w/w) of genisteins, and 10 to 50%(w/w), preferably 15 to 35%(w/w), or more preferably 25 to 35% (w/w) of glyciteins, as a percentage of the sum total weight of all isoflavones contained in the equol-containing soybean hypocotyl fermented product. The amount of saponin contained in the equol-containing soybean hypocotyl fermented product is for example 10 to 80 mg, preferably 20 to 50 mg, or more preferably 30 to 40 mg per 1 g (dry weight) of the soybean hypocotyl fermented product.

Some of the microorganisms having the ability to produce equol as described above have the ability to convert arginine to ornithine, and they can be selected from Lactococcus garvieae. An example thereof is Lactococcus 20-92 (FERM BP-10036). When soybean hypocotyls are fermented with such microorganisms, an equol-containing soybean hypocotyl fermented product containing ornithine can be obtained by adding arginine to and then fermenting the soybean hypocotyls. The amount of ornithine contained in such an equol-containing soybean hypocotyl fermented product is for example 0.5 to 2 mg, preferably 8 to 15 mg, or more preferably 9 to 12 mg per 1 g (dry weight) of the soybean hypocotyl fermented product. Such an equol-containing soybean hypocotyl fermented product can also exhibit useful physiological activities based on ornithine.

When the equol-containing soybean hypocotyl fermented product is used in the present invention, the equol-containing soybean hypocotyl fermented product may be used as it is after fermentation, or may be subjected to heat treatment, if necessary, and used in the dry solid form thus obtained. Form the point of view of preservation stability, the equol-containing soybean hypocotyl fermented product is preferably subjected to heating and drying treatment till it becomes a solid. The heated and dried equol-containing soybean hypocotyl fermented product may be subjected to powderization treatment till it becomes powder, if necessary.

In the present invention, the daily intake of equol is 0.1 to 50 mg, preferably 0.5 to 30 mg, more preferably 1 to 20 mg, depending on the degree of a symptom to be ameliorated. The amount of equol contained in the ameliorator of the present invention can be appropriately determined within the range of the daily intake of equol, considering a dosage form etc. of the ameliorator of the present invention. One or plural ameliorators of the present invention may be ingested so that the daily intake is reached.

In the present invention, the daily intake of the equol-containing soybean hypocotyl fermented product is for example 0.005 to 5 g, preferably 0.025 to 30 g, more preferably 1 to 20 g, depending on the degree of a symptom to be ameliorated.

A combination ratio (total weight ratio) of γ-tocopherol to equol contained in the ameliorator of the present invention comprising γ-tocopherol and equol is not particularly limited, and is for example 1:0.0001 to 1:500, preferably 1:0.0006 to 1:0.6, or more preferably 1:0.002 to 1:0.3.

The ameliorator of the present invention may comprise other components that do not impair the ameliorating effect on female-specific physical and/or mental unpleasant symptoms. Examples of the other components include nutrient enhancers such as vitamins, and minerals (for example, potassium, calcium, magnesium, ferrum, zinc, etc.); excipients such as lactose, starch, cellulose, maltitol, and dextrin; surfactants such as glycerin fatty acid ester, and sucrose fatty acid ester; coating agents such as gelatin, shellac, and zein; oils such as wheat germ oil, rice germ oil, and sunflower oil; wax such as beeswax, and rice bran wax; sweeteners such as sucrose, glucose, fructose, stevia, saccharin, and sucralose; and acidulants such as citric acid, malic acid, and gluconic acid, and they can be contained as appropriate.

The ameliorator of the present invention may further comprise various additives such as a solvent, a dispersant, an emulsifier, a stabilizer, a buffer, a filler, a binder, a disintegrant, a lubricant, etc.

When the ameliorator of the present invention contains calcium, the amount of calcium is not particularly limited, and the daily intake of calcium is for example 0.1 to 2500 mg, preferably 50 to 650 mg, or more preferably 100 to 300 mg. The amount of calcium contained in the ameliorator of the present invention can be appropriately determined within the range of the daily intake of calcium.

When the ameliorator of the present invention contains ferrum, the amount of ferrum is not particularly limited, and the daily intake of ferrum is for example 0.1 to 40 mg, preferably 0.2 to 15 mg, or more preferably 0.5 to 10 mg. Typically, in the present invention, the daily intake of ferrum is about 4 mg. The amount of ferrum contained in the ameliorator of the present invention can be appropriately determined within the range of the daily intake of ferrum.

The ameliorator of the present invention is utilized as a medicament, which can be in the form of a beverage or food product, or a composition for food. As used herein, the composition for food means a material for beverage or food, a food additive and the like which are used for production of a beverage or food product. The ameliorator of the present invention can be also utilized, in a non-claimed aspect, as a beverage or food product such as a food with functional claims, a food for specified health use, a health food, a nutritional supplement (supplement), a medical food, etc.

The ameliorator of the present invention can take the form of a usual medicament or a beverage or food product. Examples of the form include a tablet, a granule, a capsule (for example, soft capsule or hard capsule), powder, liquid, suspension, an effervescent agent, a chewable tablet, confectionary or snack (for example, cookie, biscuit, chocolate confection, chips, cake, gum, candy, gummy candy, steamed bean-jam bun (MANJUU), azuki-bean jelly (YOUKAN), pudding, jelly, yogurt, ice cream, sherbet, etc.), bread, noodle, rice dish, cereal, beverage (for example, refreshing beverage, carbonated beverage, energy drink, powdered drink, fruit beverage, milk beverage, jelly beverage, etc.), soup (for example, powdered soup, freeze-dried soup, retort pouch soup, etc.), bean paste soup (miso soup) (for example, powdered miso soup, freeze-dried miso soup, etc.), etc. The ameliorator of the present invention can be produced in any form as described above by a conventional method.

In the ameliorator of the present invention comprising γ-tocopherol and equol, the γ-tocopherol and the equol may be contained as distinct preparations from each other or in the same preparation.

When the ameliorator of the present invention comprising γ-tocopherol and equol comprises the γ-tocopherol and the equol as distinct preparations from each other, the respective agents may have the same form or may have different forms. For example, the ameliorator of the present invention comprising γ-tocopherol and equol may comprise a combination of a capsule containing γ-tocopherol and a tablet containing equol. For example, the ameliorator of the present invention comprising γ-tocopherol and equol may be a combination of a tablet containing γ-tocopherol and a tablet containing equol, or may be a tablet containing γ-tocopherol and equol. For example, the ameliorator of the present invention comprising γ-tocopherol and equol may be a beverage or food product containing γ-tocopherol and equol together.

The ameliorator of the present invention comprises γ-tocopherol and equol as effective components. A combination use of γ-tocopherol and equol is advantageous because it gives the effects of both the effective components.

The ameliorator of the present invention is ingested, for example, once a day to several times a day. A period for ingestion of the ameliorator of the present invention is not particularly limited. For example, the ameliorator of the present invention may be ingested for several days to several weeks, or for a month to several months. For example, the ameliorator of the present invention may be ingested when an unpleasant symptom appears between a premenstrual period and the end of a menstrual period, or may be ingested prophylactically before an unpleasant symptom appears. The ameliorator of the present invention may be ingested intermittently or continuously during the ingestion period as described above.

The intake amount of the ameliorator of the present invention is not particularly limited as long as the daily intake of each component can be ingested. When the ameliorator of the present invention comprises γ-tocopherol and equol and the γ-tocopherol and the equol are present as distinct preparations from each other, the agent containing γ-tocopherol and the agent containing equol may be ingested at the same time or may be separately ingested at some interval.

A packaging form of the ameliorator of the present invention is not particularly limited. The ameliorator of the present invention may be packed in any form that is usually used for pharmaceuticals or beverage or food products. For example, the ameliorator of the present invention may be packed using a PTP sheet, aluminum foil, various films, a box, a pouch, a PET bottle, a can, a bottle, a paper container, etc. A portable packaging form is preferred so that the ameliorator of the present invention can be readily taken. For example, in the case of the ameliorator of the present invention comprising γ-tocopherol and equol, when the γ-tocopherol and the equol are present as distinct preparations from each other, the respective agents may be packed separately or together. It is preferable that one agent or plural agents together are packed into a package so that the package contains the daily intakes of the respective components of the ameliorator of the present invention. Further, a plurality of the packages each of which contains the daily intakes of the respective components may be packed, for example, so as to provide one to several weeks' worth of the ameliorator of the present invention.

In the case of the ameliorator of the present invention comprising γ-tocopherol and equol, for example, a set of one capsule containing the daily intake of γ-tocopherol and two tablets containing the daily intake of equol may be packed in a pouch. For example, 7 days' worth of the pouches (7 pouches) each of which contains the above-described set may be further packed in a package (for example, a box), or 30 days' worth of the pouches (30 pouches) may be further packed in a package. In the case that the ameliorator of the present invention takes the form of a beverage or food product, the beverage or food product may be packed into a potable package in amounts corresponding to the daily intakes of γ-tocopherol and equol.

The "female-specific physical and/or mental unpleasant symptoms" ameliorated by the ameliorator of the present invention are various unpleasant symptoms before and/or during menstruation a cause of which is believed to be fluctuation of female hormones and the like, and include premenstrual syndrome ("PMS"), physical and/or mental unpleasant symptoms during menstruation including menstrual cramps, and unpleasant symptoms caused menstrual disorder, abnormal vaginal bleeding, and the like. Specific examples of the female-specific physical and/or mental unpleasant symptoms include unpleasant symptoms such as sleepiness, low back pain, lower abdominal pain, headache, easy fatigability, shoulder stiffness, swelling, breast fullness, vertigo, rough skin, acne, melancholy, anxiousness, feeling of malaise, lethargy, impaired ability to concentrate, getting annoyed, getting irritable, nausea, stomachache, anorexia, diarrhea, etc.

Further, the "female-specific physical and/or mental unpleasant symptoms" ameliorated by the ameliorator of the present invention can be categorized into fluid retention, negative emotion, pain, concentration ability, and change in behavior power. As used herein, the "fluid retention" means various symptoms including body weight gain, rough skin (for example, acne), breast fullness, swelling (for example, abdomen, breast, leg), etc. As used herein, the "negative emotion" means various symptoms including feeling like crying, getting lonely, anxiousness, restiveness, getting annoyed, getting irritable, getting upset, melancholy, getting easily nervous, etc. As used herein, the "pain" means symptoms including getting stiff shoulders or neck, headache, lower abdominal pain, low back pain, easy fatigability, body pain, etc. As used herein, the "concentration ability" means symptoms relating to insomnia, forgetfulness, trouble thinking, poor judgment, a decrease in concentration, getting distracted, making an unexpected mistake, slow movement, etc. As used herein, the "change in behavior power" means loss of patience with study or work, taking a nap, getting lazy, avoidance of socializing with people, a decrease in efficiency of study or work, etc.

As used herein, the "amelioration" includes alleviation, prevention and treatment of the above-described unpleasant symptoms. Thus, the present invention also provides an alleviator, preventive and/or remedy for a female-specific physical and/or mental unpleasant symptom, comprising γ-tocopherol and equol, as defined in the claims.

Further, in the present invention, it was found that γ-tocopherol not only has an ameliorating effect on the fluid retention, but also ameliorates the physical and/or mental unpleasant symptoms such as the pain, the negative emotion, the concentration ability, and the change in behavior power. Thus, in a non-claimed aspect, the present disclosure provides an ameliorator of a female-specific physical and/or mental unpleasant symptom, comprising γ-tocopherol; a method of ameliorating a female-specific physical and/or mental unpleasant symptom, comprising administering γ-tocopherol; use of γ-tocopherol for ameliorating a female-specific physical and/or mental unpleasant symptom; and use of γ-tocopherol for production of an ameliorator of a female-specific physical and/or mental unpleasant symptom. As a further non-claimed aspect, the present invention provides an alleviator, preventive and/or remedy for a female-specific physical and/or mental unpleasant symptom, comprising γ-tocopherol; a method of alleviating, preventing and/or treating a female-specific physical and/or mental unpleasant symptom, comprising administering γ-tocopherol; use of γ-tocopherol for alleviating, preventing and/or treating a female-specific physical and/or mental unpleasant symptom; and use of γ-tocopherol for production of an alleviator, preventive and/or remedy for a female-specific physical and/or mental unpleasant symptom.

Further, in the present invention, it was found that equol also ameliorates the physical and/or mental unpleasant symptoms such as the pain, the negative emotion, the concentration ability, and the change in behavior power. Thus, in a non-claimed aspect, the present disclosure provides an ameliorator of a female-specific physical and/or mental unpleasant symptom, comprising equol; a method of ameliorating a female-specific physical and/or mental unpleasant symptom, comprising administering equol; use of equol for ameliorating a female-specific physical and/or mental unpleasant symptom; and use of equol for production of an ameliorator of a female-specific physical and/or mental unpleasant symptom. As a further non-claimed aspect, the present invention provides an alleviator, preventive and/or remedy for a female-specific physical and/or mental unpleasant symptom, comprising equol; a method of alleviating, preventing and/or treating a female-specific physical and/or mental unpleasant symptom, comprising administering equol; use of equol for alleviating, preventing and/or treating a female-specific physical and/or mental unpleasant symptom; and use of equol for production of an alleviator, preventive and/or remedy for a female-specific physical and/or mental unpleasant symptom.

As used herein, terms other than particularly defined or explained terms should be interpreted as meanings usually used in the fields of pharmaceuticals and food.

### EXAMPLES

Hereinafter, the present invention is explained by means of Examples which the present invention should not be limited to. Content amounts indicated in Examples means the amount contained in one dosage form.

### Example 1: Soft capsule + Tablet

A soft capsule that contains 360 mg of γ-tocopherol, and a tablet that contains a soybean hypocotyl fermented product containing 1 mg of equol and 125 mg of calcium were prepared using appropriate amounts of a capsule base, excipients, and the like by a conventional method. The equol-containing soybean hypocotyl fermented product was produced by a method as described in WO2007/066655. One capsule and two tablets made one set.

### Test Example 1: Administration test of γ-tocopherol alone (reference)

Twenty-five people who had fluid retention before menstruation were subjected to randomly-assigned placebo-controlled double-blind tested group comparative study. The subjects continuously ingested 4 capsules per day of a soft capsule containing 90 mg of γ-tocopherol as a test substance (i.e., the daily γ-tocopherol intake was 360 mg) or 4 capsules per day of a placebo capsule, twice a day in the morning and night, two capsules each time, for 7 days from 10 to 7 days before the start of menstruation. An evaluation period is between the morning of the 6th day after the start of the continuous ingestion and the morning of the 8th day after the end of the continuous ingestion. Evaluation by questionnaires was conducted before every meal. An average value of variations from a start value of the evaluation was obtained to compare the tested group with the placebo group.

The evaluation by questionnaires was conducted by VAS (visual analog scale) and MDQ (menstrual distress questionnaire). The questionnaires are shown in Table 1 and Table 2.

For VAS, the subjects indicated their symptoms about each questionnaire on a scale of "0 mm" (no symptom) to "100 mm" (the symptom is very strong/the symptom affects daily living), and an average value of variations from a start value of the evaluation was calculated. For MDQ, the subjects answered each questionnaire on a six-point scale of "0" (no symptom) to "5" (the symptom is very strong/the symptom affects daily living). The questionnaires were divided into 8 categories as shown in Table 2, and a total of scores was calculated.

**[Table 1]**

| |
|---|
| VAS (0-100 mm) |
| Facial swelling |
| Swelling or fullness of arm or hand |
| Breast fullness or pain |
| Fullness of lower abdomen or around waist |
| Leg swelling |
| Tired or heavy feeling in the legs |
| Heavy feeling in the body |
| General sick feeling |
| Annoyance or irritability |
| Gluttonous appetite |

**[Table 2]**

| MDQ (score 0-5) | | | |
|---|---|---|---|
| Fluid retention | Body weight gain | Pain | Shoulders or neck stiffness |
| | Rough skin | | Headache |
| | Breast pain or fullness | | Lower abdominal pain |
| | Swelling (abdomen, breast, leg) | | Low back pain |
| Negative emotion | Feeling like crying at anything | | Easy fatigability |
| | Getting lonely | | Body pain |
| | Anxiousness | Concentration ability | Insomnia |
| | Restiveness | | Easy forgetfulness |
| | Getting annoyed or irritable | | Trouble thinking |
| | Getting upset | | Poor judgment |
| | Melancholy | | Decrease in concentration |
| | Getting easily nervous | | Getting distracted |
| Elevation of emotion | Being in a kindly mood | | Making an unexpected mistake |
| | Being honest | | Slow movement |
| | Getting excited easily | Change in behavior | Loss of patience with study or work |
| | Feeling happy | | Taking a nap |
| | Getting active | | Getting lazy |
| Control | Being hard to breathe | | Avoidance of socializing with people |
| | Feeling chest constriction | | Decrease in efficiency of study or work |
| | Tinnitus | Autonomic dystonia | Vertigo |
| | Palpitation | | Cold sweat |
| | Numbness in limb | | Nausea |
| | Blurred vision or bleariness | | Facial glow |
| Other | Change of food preference | Total | 47 items |

### Result 1 of Test Example 1: VAS

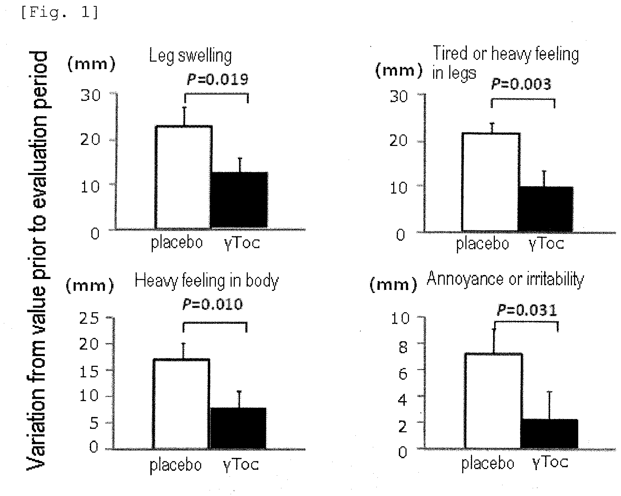

Results of the administration test of γ-tocopherol alone are shown in Figure 1. The administration test of γ-tocopherol alone shows proportions of people who had decreased scores as compared with placebo-administered people. In a γ-tocopherol-ingested group, ingestion of γ-tocopherol alone resulted in significant amelioration of not only physical unpleasant symptoms such as leg swelling but also mental unpleasant symptoms of "annoyance or irritability" as compared with a placebo group.

### Result 2 of Test Example 1: MDQ

As a result of the administration test of γ-tocopherol alone, proportions of people in the γ-tocopherol-ingested group who had decreased scores as compared with the placebo group were 56.0% for item "fluid retention", 44.0% for item "pain", 28.0% for item "negative emotion", 36.0% for item "concentration ability", and 32.0% for "change in behavior", showing high ameliorating rates.

Thus, it was found that ingestion of γ-tocopherol alone ameliorates not only physical unpleasant symptoms such as "fluid retention" and "pain" but also mental unpleasant symptoms such as "annoyance or irritability", "negative emotion", "concentration ability" and "change in behavior".

### Test Example 2: Combined administration test of γ-tocopherol and equol

Twenty-five people who had any unpleasant symptom before menstruation were subjects. The subjects continuously ingested test substances (a soft capsule containing 360 mg of γ-tocopherol as the daily intake (reference), and two tablets each of which contained a soybean hypocotyl fermented product containing 1 mg of equol, 2 mg of ferrum and 125 mg of calcium (the daily intakes of equol, ferrum and calcium were 2 mg, 4 mg and 250 mg respectively) once a day for 7 days from 10 to 7 days before the expected start of menstruation.

Before ingestion of the test substances and after the continuous ingestion for 7 days, symptoms before menstruation were evaluated by MDQ. MDQ was conducted as described in Test Example 1. Degrees of general amelioration before and during menstruation were evaluated on three-point scale of "the symptom was milder than usual", "the symptom was the same as usual", and "the symptom was worse than usual". In addition, questionnaire surveys about premenstrual symptoms before and after ingestion of the test substances, and symptoms during menstruation after the continuous ingestion were conducted.

### Result 1 of Test Example 2: MDQ

For 11 subjects of the 25 subjects, MDQ was conducted before menstruation, and proportions of people who had decreased scores as compared with before ingestion of the test substances were calculated. Regarding the other 14 subjects, MDQ could not be conducted before menstruation because menstruation started midway.

When the test substances comprising γ-tocopherol and equol were ingested, proportions of people who had decreased scores as compared with before the ingestion were 54.5% for item "fluid retention", 63.6% for item "pain", 60.0% for item "negative emotion", 54.5% for item "concentration ability", and 60.0% for "change in behavior".

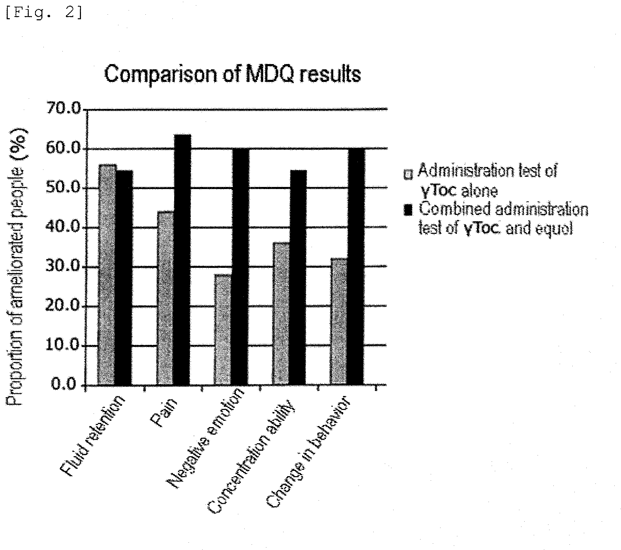

Figure 2 shows comparison of MDQ between the result 2 of Test Example 1 and the result 1 of Test Example 2. Particularly, regarding the four categories of "pain", "negative emotion", "concentration ability" and "change in behavior", administration of the combination of γ-tocopherol and equol increased the proportions of people who had ameliorated symptoms as compared with administration of γ-tocopherol alone. Further, the results of Test Example 1 and Test Example 2 suggest that ingestion of equol alone has effects on "pain", "negative emotion", "concentration ability" and "change in behavior".

### Result 2 of Test Example 2: Degrees of general amelioration

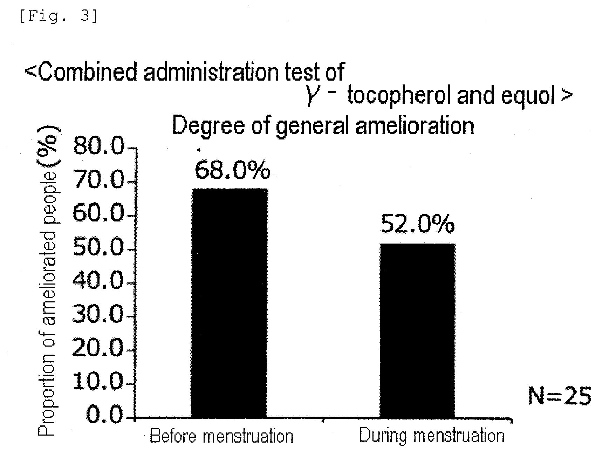

Regarding the 25 subjects, degrees of general amelioration of symptoms before and during menstruation by ingestion of the test substances were evaluated at a three-point scale of "the symptom was milder than usual", "the symptom was the same as usual", and "the symptom was worse than usual". Results are shown in Figure 3. As clear from Figure 3, ingestion of the combination of γ-tocopherol and equol ameliorated unpleasant symptoms before and during menstruation in more than half of the subjects (68.0% before menstruation; 52.0% during menstruation).

### Result 3 of Test Example 2: Results of questionnaires

According to results of the questionnaire surveys, some of the subjects did not undergo rough skin before and during menstruation, and did not suffer from acne which had always occurred before menstruation.

Thus, it was found that ingestion of the combination of γ-tocopherol and equol produces ameliorating effects on female-specific physical and/or mental unpleasant symptoms, in particular physical and/or mental unpleasant symptoms before and/or during menstruation, as compared with ingestion of γ-tocopherol alone.

## Claims

1. An ameliorator for use in a female-specific physical and/or mental unpleasant symptom before and/or during menstruation, comprising γ-tocopherol and equol, which is to be ingested so as to provide a daily γ-tocopherol intake of 0.1 to 1000 mg and a daily equol intake of 0.1 to 50 mg, respectively.

2. The ameliorator for use according to claim 1, which comprises the γ-tocopherol and the equol as distinct preparations from each other.

3. The ameliorator for use according to claim 1, which comprises the γ-tocopherol and the equol in the same preparation.

4. The ameliorator for use according to anyone of claims 1 to 3, wherein the female-specific physical and/or mental unpleasant symptom before and/or during menstruation is at least one selected from pain, negative emotion, concentration ability, and change in behavior.

5. The ameliorator for use according to any one of claims 1 to 4, wherein a combination ratio (total weight ratio) of the γ-tocopherol to the equol is 1:0.0001 to 1:500.

6. The ameliorator for use according to any one of claims 1 to 5, which has the form of a beverage or food product.

7. A beverage or food product comprising the ameliorator for use according to any one of claims 1 to 5.

## Patentansprüche

1. Linderungsmittel zur Verwendung bei einem frauenspezifischen körperlichen und/oder geistigen unangenehmen Symptom vor und/oder während der Menstruation, das γ-Tocopherol und Equol umfasst und das so einzunehmen ist, dass man eine tägliche γ-Tocopherol-Aufnahme von 0,1 bis 1000 mg und eine tägliche Equol-Aufnahme von 0,1 bis 50 mg erhält.

2. Linderungsmittel zur Verwendung gemäß Anspruch 1, das das γ-Tocopherol und das Equol als unterschiedliche Präparate umfasst.

3. Linderungsmittel zur Verwendung gemäß Anspruch 1, das das γ-Tocopherol und das Equol in demselben Präparat umfasst.

4. Linderungsmittel zur Verwendung gemäß einem der Ansprüche 1 bis 3, wobei es sich bei dem frauenspezifischen körperlichen und/oder geistigen unangenehmen Symptom vor und/oder während der Menstruation um wenigstens eines handelt, das aus Schmerzen, negativen Gefühlen, Konzentrationsfähigkeit und verändertem Verhalten ausgewählt ist.

5. Linderungsmittel zur Verwendung gemäß einem der Ansprüche 1 bis 4, wobei das Kombinationsverhältnis (Gesamtgewichtsverhältnis) des γ-Tocopherols zu dem Equol 1:0,0001 bis 1:500 beträgt.

6. Linderungsmittel zur Verwendung gemäß einem der Ansprüche 1 bis 5, das die Form eines Getränks oder Lebensmittels aufweist.

7. Getränk oder Lebensmittel, das das Linderungsmittel zur Verwendung gemäß einem der Ansprüche 1 bis 5 umfasst.

## Revendications

1. Agent d'amélioration destiné à être utilisé dans le cas d'un symptôme désagréable, physique et/ou mental, spécifique à la femme avant et/ou pendant les règles, comprenant du γ-tocophérol et de l'équol, qui doit être ingéré afin de fournir respectivement une dose journalière de γ-tocophérol de 0,1 à 1000 mg et une dose journalière d'équol de 0,1 à 50 mg.

2. Agent d'amélioration destiné à être utilisé selon la revendication 1, qui comprend le γ-tocophérol et l'équol sous forme de préparations distinctes l'une de l'autre.

3. Agent d'amélioration destiné à être utilisé selon la revendication 1, qui comprend le γ-tocophérol et l'équol dans la même préparation.

4. Agent d'amélioration destiné à être utilisé selon l'une quelconque des revendications 1 à 3, où le symptôme désagréable, physique et/ou mental, spécifique à la femme avant et/ou pendant les règles est au moins un symptôme choisi parmi la douleur, une émotion négative, la faculté de concentration et un changement de comportement.

5. Agent d'amélioration destiné à être utilisé selon l'une quelconque des revendications 1 à 4, où un rapport de combinaison (rapport pondéral total) entre le γ-tocophérol et l'équol est de 1:0,0001 à 1:500.

6. Agent d'amélioration destiné à être utilisé selon l'une quelconque des revendications 1 à 5, qui se présente sous la forme d'une boisson ou d'un produit alimentaire.

7. Boisson ou produit alimentaire comprenant l'agent d'amélioration destiné à être utilisé selon l'une quelconque des revendications 1 à 5.
